# EUROPEAN PATENT SPECIFICATION

(11) **EP 0 543 811 B1**
(45) Date of publication and mention of the grant of the patent: **12.07.2000**
(21) Application number: 90913953.7
(22) Date of filing: 17.09.1990
(51) Int. Cl.: A61K 31/415, A61K 9/06

(54) **TOPICAL TREATMENT OF BLEPHARITIS**
TOPISCHE BEHANDLUNG VON BLEPHARITIS
TRAITEMENT LOCAL DE LA BLEPHARITE

(30) Priority: 16.08.1990 US 568461
(43) Date of publication of application: 02.06.1993
(73) Proprietor: Bloom, Leonard, Towson Maryland 21204 (US); TOWSEND, Marvin S, Towson, MD 21204 (US); MARTIN, Neil F., Potomac, MD 20854 (US); ROBINSON, Howard N., Lutherville, MD 21093 (US)
(72) Inventor: MARTIN, Neil, F., Potomac, MD 20854 (US); ROBINSON, Howard, N., Lutherville, MD 21093 (US)
(74) Representative: Eddowes, Simon
(86) International application number: US9005173
(87) International publication number: WO9203133

(56) References cited:
- WO-A-89/06537
- FR-A- 2 333 500
- FR-A- 2 624 736
- US-A- 4 612 193
- Arch Dermatol, volume 123, May 1987, P.A. Bleicher et al.: "Topical metronidazole therapy for rosacea", pages 609-614
- Survey of Ophthalmology, volume 31, no. 3, November/December 1986, D.J. Browning et al.: "Ocular Rosacea", pages 145-158
- Chemotherapy, volume 29, 1983, A. Karger AG, (Basel, CH), J. Mattila et al.: "Penetration of metronidazole and trinidazole into the aqueous humor in man", pages 188-191

## Description

### Technical Field

The present invention relates to the field of treating abnormal eye inflammation and more particularly to the topical treatment of inflammations and other dysfunctions of the eyelid and conjunctiva. The present invention is especially concerned with the treatment of meibomian gland dysfunction, blepharitis, and blepharoconjunctivitis particularly associated with ocular rosacea.

### Art

Blepharitis is an inflammation of the eyelids. Blepharoconjunctivitis is an inflammation of the eyelids and the conjunctiva of the eye. Both conditions are associated with the condition known as ocular rosacea.

Rosacea is a disease of the skin (acne rosacea) and eyes (ocular rosacea) of unknown etiology and a variety of manifestations. The clinical and pathological features of the eye disease are nonspecific, and the disease is widely underdiagnosed by ophthalmologists.

More specifically with respect to ocular rosacea, ocular rosacea may involve the eyelids, conjunctiva, and cornea. Common manifestations of ocular rosacea include blepharitis, blepharoconjunctivitis, meibomianitis, chalazia, styes and conjunctival hyperemia.

References which discuss ocular rosacea include: "Ocular Rosacea" by M.S. Jenkins et al, American Journal of Ophthalmology, Vol. 88:618-622 (1979); "Blepharitis Associates With Acne Rosacea and Seborrheic Dermatitis" by J. P. McCulley et al, in Oculocutaneous Diseases, edited by J. P. Callen et al, Little, Brown & Company, International Ophthalmology Clinics, Spring 1985, Vol. 25, No. 1, pp. 159-172; and "Ocular Rosacea" by D. J. Browning et al, Survey of Ophthalmology, Vol. 31, No. 3, November-December 1986, pp. 145-158.

In the article by McCulley et al mentioned above, on pages 170-172, several treatments for blepharitis are disclosed. These treatments include: topical antibiotics; oral tetracycline; SSA neutralizers; exoenzymatic inhibitors; vitamin A analogs; and other means of affecting meibomian gland secretions.

In another prior art reference, Textbook of Dermatology, 4th Edition, A. Rook et al editors, Vol. 2, p. 3252, there is a disclosure that Demodectic blepharitis may be treated with bathing with boric acid or with benzalkonium chloride.

In the article by Browning et al mentioned above, on p. 155, there is a disclosure that for treatment of ocular rosacea only tetracycline has been critically studied. In the sane article, there is mentioned that metronidazole has been used for treatment of skin lesions of rosacea. However, the article does not teach the use of a nitroimidazole compound (including metronidazole) with a suitable carrier for topical treatment of ocular tissues.

In another reference, namely "Topical Metronidazole Therapy for Rosacea", by P. A. Bleicher et al, Arch Dermatol., Vol. 123, May 1987, pp. 609-614, there is a disclosure that metronidazole can be used in a gel for treatment of rosacea of the skin. However, there is no disclosure that metronidazole can be used for ocular rosacea.

The prior art also teaches other treatments for eye inflammations using the direct application of a treating composition to the eye. For example, in U.S. Patent 4,612,193 to Gordon et al, there is a disclosure that a blepharitic infection (not characterized as being caused by ocular rosacea) can cause a stye and that an ointment is provided to treat the stye. The ointment is based on yellow mercuric oxide, boric acid, and wheat germ oil.

In the book Diseases of the Cornea, 2nd Edition, by M.G. Grayson, C. Z. Mosby Company, 1983, pp. 119-209, there is a disclosure that blepharitis can be treated using antibiotic ointments containing antibiotics such as bacitracin, erythromycin, chloramphenicol, and tetracycline. Other active agents for treating blepharitis include Rifampin, a very dilute steroid such as 0.12%, prednisolone, and polysulfide.

The prior art treatments for eye inflammations have several disadvantages. For example, when tetracycline is taken orally it takes between two to three months to have a significant effect. Furthermore, tetracycline is plagued with side effects such as super infections, light sensitivity, cramp feelings of the user, contraindication if the user is pregnant, and resultant feelings that are similar to those when a person has the flu. Therefore, it would be desirable to avoid the use of tetracycline for the treatment of eye inflammations (e.g. ocular rosacea and related conditions).

Another eye condition is known as dry eye which results from an abnormal deficiency of tear production. A discussion of dry eye is found in the article entitled "Tear Physiology and Dry Eyes" by F. J. Holly et al, Survey of Ophthalmology, Vol. 22, No. 2, September-October 1977, pp. 69-87. As disclosed in the Holly et al article, the primary treatment for dry eye is the use of artificial tears applied topically. Unfortunately, blepharitis is often misdiagnosed as dry eye. As a result, treatment with artificial tears is inadequate to cure the patient's problem. It would be desirable to provide a pharmaceutical composition that would treat the actual blepharitis in the instance where the condition was misdiagnosed as dry eye.

Another problem that has received attention in the ophthalmological literature lately is infection by a parasite known as Acanthamoeba hystolytica which particularly plagues users of contact lenses. A particularly devastating infection results from this parasite leaving the victim particularly susceptible to blindness in an infected eye. A presently used treatment for Acanthamoeba is a therapeutic agent known as brolene which is an over-the-counter British stye medication. Other known treatments for Acanthamoeba include antibiotics such as micadasol and mediasforan. However, it would be desirable if another non-antibiotic agent could be applied topically to alleviate the deleterious conditions caused by the Acanthamoeba organism.

Another problem associated with wearers of contact lenses is the formation of film and deposits on the surface of the lenses. Lumpy deposits formed on the contact lenses are very often due to undiagnosed blepharitis. By alleviating the underlying blepharitis condition, the cause of deposit formation on contact lenses could be alleviated or removed. In this respect, it would be desirable to provide a treatment to prevent deposit formation on contact lenses that result from undiagnosed blepharitis.

The aforementioned contact lens surface film and deposits caused by blepharitis are associated with the formation of bumps (giant papillae) under the upper lids of contact lens wearers. This condition, giant papillary conjunctivitis (GPC), is an immune-mediated reaction to contact lens surface contamination and may require patients to discontinue contact lens wear. By alleviating the underlying blepharitis condition, the cause of the contamination which leads to GPC could be alleviated. This treatment may then allow certain wearers of contact lenses to resume use.

Still another option of the invention is to provide a treatment to prevent the development of the condition known as giant papillary conjunctivitis (GPC).

Although systemic treatments for eye conditions are known, such treatments are not popular with ophthalmologists. An eye doctor generally prefer to prescribe an eye medicine that is administered topically to the eye rather than prescribe a pill or the like which administers the medicine systemically. Therefore, it would be desirable to provide a treatment for blepharitis, or blepharoconjunctivitis, or ocular rosacea generally that is administered in a form such as a topically applied ointment or topically applied drops.

### Disclosure of Invention

Accordingly, it is an object of the present invention to alleviate the disadvantages and deficiencies of the prior art by providing a treatment for meibomian gland dysfunction, blepharitis, blepharoconjunctivitis, and ocular rosacea that is administered in the form of eye drops or other topically administered eye preparations.

Another object of the invention is to provide a treatment that avoids the use of systemic tetracycline or other systemic antibiotics for treating ocular inflammations such as ocular rosacea and related conditions.

Another object of the invention is to provide a pharmaceutical composition that treats actual blepharitis in an instance where the actual condition is misdiagnosed as dry eye.

Still another object of the invention is to provide a topical treatment for the eye conditions resulting from infection by Acanthamoeba hystolytica.

Yet another object of the invention is to provide a treatment to prevent deposit formation on contact lenses that result from undiagnosed blepharitis and related giant papillary conjunctivitis (GPC).

In one aspect, the invention provides the use of a nitroimidazole compound in the manufacture of a topical eye preparation for the treatment of blepharitis and/or blepharoconjunctivitis and/or ocular rosacea by topical application of the preparation to ocular tissues.

The present invention therefore relates to a pharmaceutical composition for treating blepharitis and blepharoconjunctivitis generally and especially associated with ocular rosacea. The pharmaceutical composition includes an amount of nitroimidazole compound effective for treating the blepharitis and/or blepharoconjunctivitis and/or ocular rosacea; and a carrier for the nitroimidazole compound wherein the carrier is suitable for direct application to the eye tissues. The nitroimidazole compound is selected from the group consisting of metronidazole, nimorazole, tinidazole, ordinidazole, secnidazole, and carnidazole. The preferred compound is metronidazole.

The carrier may be in the form of an ointment, e.g. petrolatum-based or a water soluble gel, or in the form of a liquid to be applied to the eye in the form of eye drops. The eye drops can be in a bottle containing a plurality of doses or can be in a unidose dispenser.

One carrier for eye drops is an artificial tear composition including primarily isotonic sodium chloride. Other artificial tear ophthalmic carriers for the eye drops can be a hypertonic composition or a hypotonic composition based on sodium chloride solution. Moreover, other hypertonic, isotonic, and hypotonic carriers can be used. In addition, a cellulose ether such as methylcellulose may be added to the artificial tear carrier. Other cellulose ethers such as hydroxypropylmethylcellulose and hydroxyethylcellulose may be included in the artificial tear carrier. The artificial tear composition may also include a polyvinyl alcohol.

Another carrier administered in the form of eye drops can have a nonaqueous liquid base, e.g. a mineral oil based carrier. For example, the liquid ocular lubricant LIPO-TEARS, which contains a blend of mineral oil and white petrolatum U.S.P., can be used. The carrier LIPO-TEARS is made by Spectra Pharmaceutical Services, Inc., Hanover, Massachusetts 02339.

Yet another carrier for ocular administration of the nitroimidazole compound is a suspension of solid particles in a liquid. More specifically, the ocular administration can be in the form of an aqueous suspension. Even more specifically, a carrier for administering the nitroimidazole in suspension form can be particles of an ion-exchange resin, e.g. Amberlite, suspended in water. Employment of the ion-exchange resin Amberlite in an aqueous suspension for ocular administration of a betaxolol HC1 treating agent (a beta-adrenergic blocker) for treating glaucoma is embodied in BETOPIC®S made by Alcon Laboratories, Inc., Fort Worth, Texas 76134.

The composition of the invention is applied to ocular tissues directly for treating the conditions of blepharitis, blepharoconjunctivitis, and ocular rosacea.

Another carrier for the nitroimidazole compound can be a shampoo formulation for the eye. In this respect, the nitroimidazole compound can be part of an eyelash shampoo or eyescrub for cleansing hair associated with the eye. Other carriers for the metronidazole compounds can be slow release inserts, aerosols, collagen shields, bandage contact lenses, and contact lens solutions.

Further aspects and embodiments of the invention are set forth in the claims.

These and other objects and advantages of the present invention will become apparent from a reading of the following specification.

### Mode for Carrying Out the Invention

Here are represented several formulations for pharmaceutical compositions of the invention.

### Example 1

One gram of metronidazole is added to 1,000 grams of artificial tear carrier with stirring. The artificial tear carrier is isotonic sodium chloride solution. This formulation provides an approximately 0.1% solution of metronidazole in artificial tear carrier for application to the patient by means of eye drops.

### Example 2

One gram of metronidazole is added to 1,000 grams of artificial tear carrier with stirring. The artificial tear carrier is hypertonic sodium chloride solution. This formulation provides an approximately 0.1% solution of metronidazole in artificial tear carrier for application to the patient by means of eye drops.

### Example 3

One gram of metronidazole is added to 1,000 grams of artificial tear carrier with stirring. The artificial tear carrier is hypotonic sodiun chloride solution. This formulation provides an approximately 0.1% solution of metronidazole in artificial tear carrier for application to the patient by means of eye drops.

### Example 4

7.5 grams of metronidazole are added to 992.5 grams of artificial tear solution with stirring to provide a formulation containing approximately 0.75% metronidazole in an artificial tear carrier.

### Example 5

10 grams of metronidazole are added to 990.0 grams of isotonic sodium chloride solution with stirring to provide a 1% metronidazole solution in isotonic sodium chloride carrier.

### Example 6

An eye drop formulation is made up by blending the following: 10 grams metronidazole, 10 grams methylcellulose, and 980 grams isotonic sodium chloride. This formulation contains approximately 1% metronidazole, 1% methylcellulose, and the balance being isotonic sodium chloride carrier.

### Example 7

Another eye drop formulation is made up by blending the following: 10 grams metronidazole, 14 grams polyvinyl alcohol, and 976 grams isotonic sodium chloride artificial tear solution. The resulting formulation contains approximately 1% metronidazole, 1.4% polyvinyl alcohol, and the balance being artificial tear carrier.

### Example 8

Another eye drop formulation is made by blending the following: 15 grams metronidazole and 985 grams of isotonic sodium chloride artificial tear solution with stirring to provide a 1.5% metronidazole solution.

### Example 9

Another eye drop formulation is made by stirring 20 grams metronidazole into 980 grams of artificial tear solution to provide a 2.0% metronidazole solution.

In addition to the artificial tear carriers disclosed above, an artificial tear carrier for the nitroimidazole compound can be selected from an artificial tear formulation selected from the formulations set out in Table I below.

**TABLE I**

| Major Component | Trade Name | Preservative |
|---|---|---|
| Hydroxyothyl-cellulose | Clerz | thimerosal + edetate disodium |
| | Lyteers | benzalkonium chloride + edetate disodium |
| | Teargard | thimersal + ededate disodium |
| Hydroxypropyl-cellulose | Lacrisert* (Water soluble insert) | benzalkonium chloride + edetate disodium |
| Hydroxpropl methycellulose | Isopto Alkaline | benzalkonium chloride |
| | Isopto Plain | benzalkonium chloride |
| | Isopto Tears | bentalkonium chloride |
| | Lacril | chlorobutanol |
| | Muro Tears | benzalkonium chloride + edetate disodium |
| | Tearisol | benzalkonium chloride + edetate disodium |
| Methylcellulose | Methopto | benzalkonium chloride |
| | Methulose | benzalkonium chloride |
| | Murocel | methylparaben & propylparaben |
| Carboxymethyl-cellulose | Celluvisc | preservative free |
| | Visculose | benzalkonium chloride |
| Polyvinyl alcohol | Aqua Tears | benzalkonium chloride + edetate sodium |
| | Liquifilm Tears | chlorobutanol |
| | Liquifilm Forte | thimerosal + edetate sodium |
| | Tears Plus | chlorobutanol |
| Polyvinyl alcohol and cellulose ester | aqua-FLOW | benzalkonium chloride + edetate disodium |
| | Neo-Tears | thimerosal + edetate disodium |
| Polyvinyl alcohol and povidone Other Polymeric Systems | Refresh | preservative free |
| | Adapettes | thimerosal & edetate disodium |
| | Adsorbotear | thimerosal & edetate disodium |
| | Comfort Drops | benzalkonium chloride + edetate disodium |
| | Dual Wet | benzalkonium chloride + edetate disodium |
| | Hypotears | benzalkonium chloride + edetate disodium |
| | Tears Naturale | benzalkonium chloride + edetate disodium |

More complete descriptions of artificial tear carriers are found in PDR for Nonprescription Drugs, 1990, pages 504-506.

Moreover, the TEARS NATURAl® is made by Alcon, (6201 South Freeway, Fort Worth, TX 76134) and is comprised of DUASORB® (Dextran 70 Hydroxypropyl Methylcellulose) as a water soluble polymeric system with preservatives benzalkonium chloride, 0.01% and Edetate Disodium 0.05% which is disclosed in U.S. Patent No. 4,039,662, incorporated herein by reference.

### Example 10

The following ointment can be prepared by blending 10 grams of metronidazole thoroughly with 990 grams petrolatum vehicle (an ointment base) to provide an ointment suitable for application to the ocular tissues which contains 1% metronidazole.

### Example 11

The following ointment can be prepared by blending 15 grams of metronidazole thoroughly with 985 grams petrolatum vehicle (an ointment base) to provide an ointment suitable for application to the ocular tissues which contains 1.5% metronidazole.

### Example 12

The following ointment can be prepared by blending 15 grams of metronidazole thoroughly with 980 grams petrolatum vehicle (an ointment base) to provide an ointment suitable for application to the ocular tissues which contains 2% metronidazole.

Other ointments based upon the petrolatum carrier can be selected from the carriers listed below in Table II.

**TABLE II**

| Trade Name | Composition |
|---|---|
| Akwa-Tears (Akorn) | Petrolatum, Liquid Lanolin, Mineral Oil |
| Duolube (Muro) | Sterile ointment containing white petroleum and mineral oil |
| Duratears (Alcon) | Sterile ointment with white petroleum, liquid lanolin, mineral oil, methylparaben and polyparaben |
| Hypotears (Cooper Vision) | Sterile ointment containing white petroleum and light mineral oil |
| Lacri-Lube S.O.P. (Allergan) | Sterile ointment with 42.5% mineral oil, 55% white petrolatum, lanolin, and chlorobutanol |

An ophthalmic gel carrier can also be used for administering the nitroimidazole compound directly to ocular tissues. A suitable ophthalmic gel carrier is comprised of approximately 10% CARBOPOL 940 (which is a synthetic high molecular weight cross-linked polymer of acrylic acid) to impart a high viscosity. A specific formulation of the invention which employs an ophthalmic gel is set forth below in Example 13.

### Example 13

An aqueous gel formulation of the invention is obtained by blending approximately 20 grams of metronidazole with approximately 980 grams of an ophthalmic gel carrier containing approximately 0.08 grams of benzalkonium chloride, 0.5 grams of Edetate Disodium, 80 grams of Carbopol 940, and approximately 900 grams of water.

Another ophthalmic gel carrier can be prepared in accordance with the teaching in U.S. Patent No. 4,788,007 incorporated herein by reference. This patent discloses an aqueous aloe vera gel. To obtain a composition for treating blepharitis in accordance with the subject invention, a quantity of metronidazole is added to a quantity of the aloe vera gel. More specifically, to obtain a gel formulation containing approximately 2% metronidazole, approximately 20 grams of metronidazole are blended with approximately 980 grams of the aloe vera gel.

As stated above, a shampoo which carries a nitroimidazole compound can be used for cleansing eyelashes and for treating ocular rosacea, blepharitis, and blepharoconjunctivitis. In Example 14 below, a formulation for an eyelash shampoo which carries the nitroimidazole compound, metronidazole, is presented.

### Example 14

The following eyelash shampoo can be prepared by adding 20 grams of metronidazole to approximately 1,000 grams of an eyelid cleanser known as I-SCRUB™ made by Spectra Pharmaceutical Services, Hanover, Massachusetts, that contains the following ingredients: PEG-200 Glyceryl Monotallowate, Disodium Laureth Sulfosuccinate, Cocoamido Propyl Amine Oxide, PEG-78 Glyceryl Monococate, Benzyl Alcohol, Disodium Edetate, and Purified Water USP. I-SCRUB™ (without the presence of the subject metronidazole) is disclosed as being suitable for hygienic care of blepharitis. With the added metronidazole, the eyelash shampoo of the invention is effective in treating the blepharitis.

By employing the principles of the invention, numerous objects are realized and numerous benefits are obtained. For example, a pharmaceutical composition is obtainable to treat blepharitis, blepharoconjunctivitis, and ocular rosacea and is administered in the form of an ointment or in the form of eye drops. The method of treatment avoids the use of tetracycline for treating ocular rosacea and related conditions. With the invention, a pharmaceutical composition is obtainable that treats actual blepharitis in the case where the condition is misdiagnosed as dry eye. The invention enables a topical treatment for eye conditions resulting from infection by Acanthamoeba hystolytica. The invention enables a treatment to prevent deposit formation on contact lenses that results from blepharitis and related giant papillary conjunctivitis.

Obviously, many modifications may be made without departing from the basic spirit of the present invention. Accordingly, it will be appreciated by those skilled in the art that within the scope of the appended claims, the invention may be practiced other than has been specifically described herein.

## Claims (Claims for the following Contracting State(s): AT, BE, CH, LI, DE, DK, FR, GB, IT, LU, NL, SE)

1. The use of a nitroimidazole compound in the manufacture of a topical eye preparation for the treatment of blepharitis and/or blepharoconjunctivitis and/or ocular rosacea by topical application of the preparation to ocular tissues.

2. The use of claim 1, wherein the medicament comprises an ophthalmic carrier suitable for topical application to ocular tissues as well as the nitroimidazole compound and the carrier comprises:
(a) a petrolatum-based vehicle or another ointment base suitable for direct application to the eye,
(b) a water soluble gel, or
(c) a liquid suitable for application to the eye in the form of drops.

3. The use of claim 1, wherein the medicament comprises an ophthalmic carrier suitable for topical application to ocular tissues as well as the nitroimidazole compound and the carrier comprises:
(a) a nonaqueous liquid base,
(b) a mineral oil base,
(c) a blend of mineral oil and petrolatum,
(d) a suspension of solid particles in a liquid, or
(e) a suspension of Amberlite or another ion-exchange resin in water.

4. The use of any of claims 1 to 3, wherein
(a) said nitroimidazole compound is present in a range of 0.1-1% and the balance is said carrier,
(b) said nitroimidazole compound is present in a range of 0.75-1% and the balance is said carrier,
(c) said nitroimidazole compound is present in a range of 0.1-2% and the balance is said carrier, or
(d) said nitroimidazole compound is present in a range of 0.75-2% and the balance is said carrier.

5. The use of any of claims 1 to 4, wherein the nitroimidazole compound is selected from metronidazole, nimoranzole, timidazole, ordinidazole, secnidazole and camidazole.

6. The use of claim 5, wherein the nitroimidazole is metronidazole.

7. A method of manufacturing a topical eye preparation for use in treating blepharitis and/or blepharoconjunctivitis and/or ocular rosacea by topical application of the preparation to ocular tissues, comprising manufacturing a nitroimidazole compound into a topical eye preparation for such use.

8. A method of claim 7, wherein an ophthalmic carrier suitable for topical application to ocular tissues is manufactured into the medicament together with the nitroimidazole compound, and the carrier comprises:
(a) a petrolatum-based vehicle or another ointment base suitable for direct application to the eye,
(b) a water soluble gel,
(c) a liquid suitable for application to the eye in the form of drops,
(d) a nonaqueous liquid base,
(e) a mineral oil base,
(f) a blend of mineral oil and petrolatum,
(g) a suspension of solid particles in a liquid, or
(h) a suspension of Amberlite or another ion-exchange resin in water.

9. A method of claim 7 or claim 8 wherein
(a) said nitroimidazole compound is present in a range of 0.1-1% and the balance is said carrier,
(b) said nitroimidazole compound is present in a range of 0.75-1% and the balance is said carrier,
(c) said nitroimidazole compound is present in a range of 0.1-2% and the balance is said carrier, or
(d) said nitroimidazole compound is present in a range of 0.75-2% and the balance is said carrier,
and/or wherein (i) the nitroimidazole compound is selected from metronidazole, nimoranzole, tinidazole, ordinidazole, secnidazole and carnidazole or (ii) the nitroimidazole compound is metronidazole.

10. A pharmaceutical composition, comprising:
an amount of nitroimidazole compound effective to treat blepharitis and blepharoconjunctivitis in an animal or human patient; and
a carrier for said nitroimidazole compound, the carrier being suitable for topical application to ocular tissues and comprising an artificial tear composition which is hypertonic, isotonic, of hypotonic.

11. A pharmaceutical composition of claim 10 wherein the artificial tear composition includes a cellulose ether or another cellulose derivative.

12. A pharmaceutical composition of claim 11 wherein the cellulose derivative is cellulose ether and the cellulose ether is present as approximately 1% by weight of the artificial tear carrier.

13. A pharmaceutical composition of claim 11 or claim 12 wherein the cellulose derivative is selected from methylcellulose, hydroxypropylmethylcellulose, and hydroxyethylcellulose.

14. A pharmaceutical composition of claim 13 wherein the cellulose derivative includes methylcellulose.

15. A pharmaceutical composition of any of claims 10 to 14 wherein the artificial tear composition includes polyvinyl alcohol.

16. A pharmaceutical composition of claim 15 wherein the polyvinyl alcohol is present as approximately 1.4% by weight of the artificial tear carrier.

17. A pharmaceutical product comprising a composition adapted for topical application to ocular tissues to the exclusion of non-ocular topical application, comprising:
an amount of nitroimidazole compound effective to treat blepharitis and blepharoconjunctivitis in an animal or human patient; and
an ophthalmic carrier for said nitroimidazole compound, said carrier being suitable for topical application to ocular tissues.

18. An eye drop preparation, eyelash shampoo, eyescrub or contact lens solution comprising a nitroimidazole compound effective in an amount to treat blepharitis and blepharoconjunctivitis in an animal or human patient.

19. A composition of claim 17 or a preparation, shampoo, eyescrub or contact lens solution of claim 18, which further includes the specific feature(s) recited in claim 4 and/or one of claims 5 and 6.

20. A product for use in the treatment of blepharitis and blepharoconjunctivitis, comprising:
a preparation which is topically administrable to ocular tissues and which comprises a nitroimidazole compound and an ophthalmically acceptable carrier,
a bottle, and
and eye drop applicator.

21. A product of claim 20 wherein the preparation further includes the specific feature(s) recited in claim 4 and/or one of claims 5 and 6.

## Claims (Claims for the following Contracting State(s): ES)

1. The use of a nitroimidazole compound in the manufacture of a topical eye preparation for the treatment of blepharitis and/or blepharoconjunctivitis and/or ocular rosacea by topical application of the preparation to ocular tissues.

2. The use of claim 1, wherein the medicament comprises an ophthalmic carrier suitable for topical application to ocular tissues as well as the nitroimidazole compound and the carrier comprises:
(a) a petrolatum-based vehicle or another ointment base suitable for direct application to the eye,
(b) a water soluble gel, or
(c) a liquid suitable for application to the eye in the form of drops.

3. The use of claim 1, wherein the medicament comprises an ophthalmic carrier suitable for topical application to ocular tissues as well as the nitroimidazole compound and the carrier comprises:
(a) a nonaqueous liquid base,
(b) a mineral oil base,
(c) a blend of mineral oil and petrolatum,
(d) a suspension of solid particles in a liquid, or
(e) a suspension of Amberlite or another ion-exchange resin in water.

4. The use of any of claims 1 to 3, wherein
(a) said nitroimidazole compound is present in a range of 0.1-1% and the balance is said carrier,
(b) said nitroimidazole compound is present in a range of 0.75-1% and the balance is said carrier,
(c) said nitroimidazole compound is present in a range of 0.1-2% and the balance is said carrier, or
(d) said nitroimidazole compound is present in a range of 0.75-2% and the balance is said carrier.

5. The use of any of claims 1 to 4, wherein the nitroimidazole compound is selected from metronidazole, nimoranzole, timidazole, ordinidazole, secnidazole and carnidazole.

6. The use of claim 5, wherein the nitroimidazole is metronidazole.

7. A method of manufacturing a topical eye preparation for use in treating blepharitis, blepharoconjunctivitis or ocular rosacea by topical application of the preparation to ocular tissues, comprising manufacturing a nitroimidazole compound into a topical eye preparation for such use.

8. A method of claim 7, wherein an ophthalmic carrier suitable for topical application to ocular tissues is manufactured into the medicament together with the nitroimidazole compound, and the carrier comprises:
(a) a petrolatum-based vehicle or another ointment base suitable for direct application to the eye,
(b) a water soluble gel,
(c) a liquid suitable for application to the eye in the form of drops,
(d) a nonaqueous liquid base,
(e) a mineral oil base,
(f) a blend of mineral oil and petrolatum,
(g) a suspension of solid particles in a liquid, or
(h) a suspension of Amberlite or another ion-exchange resin in water.

9. A method of claim 7 or claim 8 wherein
(a) said nitroimidazole compound is present in a range of 0.1-1% and the balance is said carrier,
(b) said nitroimidazole compound is present in a range of 0.75-1% and the balance is said carrier,
(c) said nitroimidazole compound is present in a range of 0.1-2% and the balance is said carrier, or
(d) said nitroimidazole compound is present in a range of 0.75-2% and the balance is said carrier,
and/or wherein (i) the nitroimidazole compound is selected from metronidazole, nimoranzole, tinidazole, ordinidazole, secnidazole and carnidazole or (ii) the nitroimidazole compound is metronidazole.

10. A method of preparing a pharmaceutical composition, comprising:
combining an amount of a nitroimidazole compound effective to treat blepharitis and blepharoconjunctivitis in an animal or human patient with a carrier for the nitroimidazole compound, the carrier being suitable for topical application to ocular tissues and comprising an artificial tear composition which is hypertonic, isotonic or hypotonic.

11. A method of claim 10, wherein the artificial tear composition includes a cellulose ether or another cellulose derivative.

12. A method of claim 11, wherein the cellulose derivative is a cellulose ether and the ether is present as approximately 1% by weight of the artificial tear carrier.

13. A method of claim 11 or claim 12, wherein the cellulose derivative is selected from methylcellulose, hydroxypropylmethylcellulose and hydroxyethylcellulose.

14. A method of claim 13, wherein the cellulose derivative includes methylcellulose.

15. A method of any of claims 10 to 14, wherein the artificial tear composition includes polyvinyl alcohol or includes polyvinyl alcohol in an amount of approximately 1.4% by weight of the artificial tear carrier.

16. A method of preparing a pharmaceutical composition, comprising combining an amount of a nitroimidazole compound effective to treat blepharitis and blepharoconjunctivitis in an animal or human patient with one of
(i) a hypotonic carrier for the nitroimidazole compound, the carrier being suitable for topical application to ocular tissues,
(ii) a hypertonic carrier for the nitroimidazole compound, the carrier being suitable for topical application to ocular tissues,
(iii) an isotonic carrier for the nitroimidazole compound, the carrier being suitable for topical application to ocular tissues, or
(iv) a carrier for said nitroimidazole compound, said carrier being suitable for topical application to ocular tissues, wherein said carrier is a shampoo suitable for cleansing eyelashes.

17. A method of making an eye drop preparation, eyelash shampoo, eyescrub or contact lens solution, comprising combining an amount of a nitroimidazole compound effective to treat blepharitis and blepharoconjunctivitis with a carrier for the nitroimidazole compound suitable for making, respectively, an eye drop preparation, eyelash shampoo, eyescrub or contact lens solution.

18. A method of claim 17, which further includes the specific feature(s) recited in claim 4 and/or one of claims 5 and 6.

19. A product comprising:
(i) a pharmaceutical preparation which is topically administrable to ocular tissues and which comprises a nitroimidazole compound in an amount effective for the treatment of blepharitis and blepharoconjunctivitis and an ophthalmically acceptable carrier; and
(ii), as non-pharmaceutical components, a bottle and an eye drop applicator.

20. A product of claim 19, which further includes the specific feature(s) recited in claim 4 and/or one of claims 5 and 6.

## Patentansprüche (Patentansprüche für folgende(n) Vertragsstaat(en): AT, BE, CH, LI, DE, DK, FR, GB, IT LU, NL, SE)

1. Verwendung einer Nitroimidazolverbindung zur Herstellung einer Zubereitung zur lokalen Anwendung am Auge zur Behandlung von Blepharitis und/oder Blepharokonjunktivitis und/oder okulärer Rosacea durch lokales Aufbringen der Zubereitung auf okuläre Gewebe.

2. Verwendung nach Anspruch 1, wobei das Arzneimittel einen opthalmischen Träger, der zum lokalen Aufbringen auf okuläre Gewebe geeignet ist sowie die Nitroimidazolverbindung umfasst, wobei der Träger umfasst:
(a) ein Vehikel auf Rohvaseline-Basis oder eine andere Salbengrundlage, die zum direkten Aufbringen auf das Auge geeignet ist,
(b) ein wasserlösliches Gel, oder
(c) eine Flüssigkeit, die zum Aufbringen auf das Auge in Form von Tropfen geeignet ist.

3. Verwendung nach Anspruch 1, wobei das Arzneimittel einen opthalmischen Träger, der zum lokalen Aufbringen auf okuläre Gewebe geeignet ist sowie die Nitrolmidazolverbindung umfasst, wobei der Träger umfasst:
(a) eine nicht-wässrige flüssige Basis,
(b) eine Mineralöl-Basis,
(c) ein Gemisch aus Mineralöl und Rohvaseline,
(d) eine Suspension von festen Teilchen in einer Flüssigkeit, oder
(e) eine Suspension von Amberlit oder einem anderen Ionenaustauscherharz in Wasser.

4. Verwendung nach einem der Ansprüche 1 bis 3, wobei
(a) die Nitroimidazolverbindung in einem Bereich von 0,1-1% vorliegt und der Rest der Träger ist,
(b) die Nitroimidazolverbindung in einem Bereich von 0,75-1% vorliegt und der Rest der Träger ist,
(c) die Nitroimidazolverbindung in einem Bereich von 0,1-2% vorliegt und der Rest der Träger ist, oder
(d) die Nitroimidazolverbindung in einem Bereich von 0,75-2% vorliegt und der Rest der Träger ist.

5. Verwendung nach einem der Ansprüche 1 bis 4, wobei die Nitroimidazolverbindung aus Metronidazol, Nimoranzol, Timidazol, Ordinidazol, Secnidazol und Carnidazol ausgewählt ist.

6. Verwendung nach Anspruch 5, wobei das Nitroimidazol Metronidazol ist.

7. Verfahren zur Herstellung einer Zubereitung zur lokalen Anwendung am Auge zur Verwendung bei der Behandlung von Blepharitis und/oder Blepharokonjunktivitis und/oder okulärer Rosacea durch lokales Aufbringen der Zubereitung auf okuläre Gewebe, umfassend Verarbeiten einer Nitroimidazolverbindung in eine Zubereitung zur lokalen Anwendung am Auge für eine solche Verwendung.

8. Verfahren nach Anspruch 7, wobei ein ophthalmischer Träger, der zum lokalen Aufbringen auf okuläre Gewebe geeignet ist, zusammen mit der Nitroimidazolverbindung in das Arzneimittel verarbeitet wird, wobei der Träger umfasst:
(a) ein Vehikel auf Rohvaseline-Basis oder eine andere Salbengrundlage, die zum direkten Aufbringen auf das Auge geeignet ist,
(b) ein wasserlösliches Gel,
(c) eine Flüssigkeit, die zum Aufbringen auf das Auge in Form von Tropfen geeignet ist,
(d) eine nicht-wässrige flüssige Basis,
(e) eine Mineralöl-Basis,
(f) ein Gemisch aus Mineralöl und Rohvaseline,
(g) eine Suspension von festen Teilchen in einer Flüssigkeit, oder
(h) eine Suspension von Amberlit oder einem anderen Ionenaustauscherharz in Wasser.

9. Verfahren nach Anspruch 7 oder 8, wobei
(a) die Nitroimidazolverbindung in einem Bereich von 0,1-1% vorliegt und der Rest der Träger ist,
(b) die Nitroimidazolverbindung in einem Bereich von 0,75-1% vorliegt und der Rest der Träger ist,
(c) die Nitroimidazolverbindung in einem Bereich von 0,1-2% vorliegt und der Rest der Träger ist, oder
(d) die Nitroimidazolverbindung in einem Bereich von 0,75-2% vorliegt und der Rest der Träger ist,
und/oder wobei (i) die Nitroimidazolverbindung aus Metronidazol, Nimoranzol, Tinidazol, Ordinidazol, Secnidazol und Carnidazol ausgewählt ist oder (ii) die Nitroimidazolverbindung Metronidazol ist.

10. Pharmazeutische Zusammensetzung, umfassend:
eine zur Behandlung von Blepharitis und Blepharokonjunktivitis in einem Tier oder einem Menschen wirksame Menge an Nitroimidazolverbindung und
einen Träger für die Nitroimidazolverbindung, wobei der Träger zum lokalen Aufbringen auf okuläre Gewebe geeignet ist und eine künstliche hypertone, isotone oder hypotone Tränenflüssigkeitszusammensetzung umfasst.

11. Pharmazeutische Zusammensetzung nach Anspruch 10, wobei die künstliche Tränenflüssigkeitszusammensetzung einen Celluloseether oder ein anderes Cellulosederivat umfasst.

12. Pharmazeutische Zusammensetzung nach Anspruch 11, wobei das Cellulosederivat Celluloseether ist und der Celluloseether zu etwa 1 Gew.-% im künstlichen Tränenflüssigkeitsträger vorliegt.

13. Pharmazeutische Zusammensetzung nach Anspruch 11 oder 12, wobei das Cellulosederivat aus Methylcellulose, Hydroxypropylmethylcellulose und Hydroxyethylcellulose ausgewählt ist.

14. Pharmazeutische Zusammensetzung nach Anspruch 13, wobei das Cellulosederivat Methylcellulose umfasst.

15. Pharmazeutische Zusammensetzung nach einem der Ansprüche 10 bis 14, wobei die künstliche Tränenflüssigkeitszusammensetzung Polyvinylalkohol umfasst.

16. Pharmazeutische Zusammensetzung nach Anspruch 15, wobei der Polyvinylalkohol zu etwa 1,4 Gew.-% im künstlichen Tränenflüssigkeitsträger vorliegt.

17. Pharmazeutisches Produkt, umfassend eine Zusammensetzung, die zum lokalen Aufbringen auf okuläre Gewebe zum Ausschluss von nicht-okulärem lokalen Aufbringen angepasst ist, umfassend:
eine zur Behandlung von Blepharitis und Blepharokonjunktivitis in einem Tier oder einem Menschen wirksame Menge an Nitroimidazolverbindung und
einen ophthalmischen Träger für die Nitroimidazolverbindung, wobei der Träger zum lokalen Aufbringen auf okuläre Gewebe geeignet ist.

18. Eine Augentropfenzubereitung, ein Wimpernshampoo, eine Augenwasch- oder Kontaktlinsenlösung, umfassend eine Nitroimidazolverbindung in einer Menge, die zur Behandlung von Blepharitis und Blepharokonjunktivitis in einem Tier oder einem Menschen wirksam ist.

19. Zusammensetzung nach Anspruch 17 oder eine Zubereitung, ein Shampoo, eine Augenwasch- oder Kontaktlinsenlösung nach Anspruch 18, weiter umfassend das/die spezielle(n) in Anspruch 4 und/oder einem der Ansprüche 5 und 6 angegebene(n) Merkmal(e).

20. Produkt zur Verwendung bei der Behandlung von Blepharitis und Blepharokonjunktivitis, umfassend:
eine Zubereitung, die lokal an okuläre Gewebe verabreichbar ist und eine Nitroimidazolverbindung sowie einen ophthalmisch verträglichen Träger umfasst,
eine Flasche und
einen Augentropfen-Applikator.

21. Produkt nach Anspruch 20, wobei die Zubereitung weiter das/die spezielle(n) in Anspruch 4 und/oder einem der Ansprüche 5 und 6 angegebene(n) Merkmal(e) umfasst.

## Patentansprüche (Patentansprüche für folgende(n) Vertragsstaat(en): ES)

1. Verwendung einer Nitroimidazolverbindung zur Herstellung einer Zubereitung zur lokalen Anwendung am Auge zur Behandlung von Blepharitis und/oder Blepharokonjunktivitis und/oder okulärer Rosacea durch lokales Aufbringen der Zubereitung auf okuläre Gewebe.

2. Verwendung nach Anspruch 1, wobei das Arzneimittel einen opthalmischen Träger, der zum lokalen Aufbringen auf okuläre Gewebe geeignet ist sowie die Nitroimidazolverbindung umfasst, wobei der Träger umfasst:
(a) ein Vehikel auf Rohvaseline-Basis oder eine andere Salbengrundlage, die zum direkten Aufbringen auf das Auge geeignet ist,
(b) ein wasserlösliches Gel, oder
(c) eine Flüssigkeit, die zum Aufbringen auf das Auge in Form von Tropfen geeignet ist.

3. Verwendung nach Anspruch 1, wobei das Arzneimittel einen opthalmischen Träger, der zum lokalen Aufbringen auf okuläre Gewebe geeignet ist sowie die Nitroimidazolverbindung umfasst, wobei der Träger umfasst:
(a) eine nicht-wässrige flüssige Basis,
(b) eine Mineralöl-Basis,
(c) ein Gemisch aus Mineralöl und Rohvaseline,
(d) eine Suspension von festen Teilchen in einer Flüssigkeit, oder
(e) eine Suspension von Amberlit oder einem anderen Ionenaustauscherharz in Wasser.

4. Verwendung nach einem der Ansprüche 1 bis 3, wobei
(a) die Nitroimidazolverbindung in einem Bereich von 0,1-1% vorliegt und der Rest der Träger ist,
(b) die Nitroimidazolverbindung in einem Bereich von 0,75-1% vorliegt und der Rest der Träger ist,
(c) die Nitroimidazolverbindung in einem Bereich von 0,1-2% vorliegt und der Rest der Träger ist, oder
(d) die Nitroimidazolverbindung in einem Bereich von 0,75-2% vorliegt und der Rest der Träger ist.

5. Verwendung nach einem der Ansprüche 1 bis 4, wobei die Nitroimidazolverbindung aus Metronidazol, Nimoranzol, Timidazol, Ordinidazol, Secnidazol und Carnidazol ausgewählt ist.

6. Verwendung nach Anspruch 5, wobei das Nitroimidazol Metronidazol ist.

7. Verfahren zur Herstellung einer Zubereitung zur lokalen Anwendung am Auge zur Verwendung bei der Behandlung von Blepharitis, Blepharokonjunktivitis oder okulärer Rosacea durch lokales Aufbringen der Zubereitung auf okuläre Gewebe, umfassend Verarbeiten einer Nitroimidazolverbindung in eine Zubereitung zur lokalen Anwendung am Auge für eine solche Verwendung.

8. Verfahren nach Anspruch 7, wobei ein ophthalmischer Träger, der zum lokalen Aufbringen auf okuläre Gewebe geeignet ist, zusammen mit der Nitroimidazolverbindung in das Arzneimittel verarbeitet wird, wobei der Träger umfasst:
(a) ein Vehikel auf Rohvaseline-Basis oder eine andere Salbengrundlage, die zum direkten Aufbringen auf das Auge geeignet ist,
(b) ein wasserlösliches Gel,
(c) eine Flüssigkeit, die zum Aufbringen auf das Auge in Form von Tropfen geeignet ist,
(d) eine nicht-wässrige flüssige Basis,
(e) eine Mineralöl-Basis,
(f) ein Gemisch aus Mineralöl und Rohvaseline,
(g) eine Suspension von festen Teilchen in einer Flüssigkeit, oder
(h) eine Suspension von Amberlit oder einem anderen Ionenaustauscherharz in Wasser.

9. Verfahren nach Anspruch 7 oder 8, wobei
(a) die Nitroimidazolverbindung in einem Bereich von 0,1-1% vorliegt und der Rest der Träger ist,
(b) die Nitroimidazolverbindung in einem Bereich von 0,75-1% vorliegt und der Rest der Träger ist,
(c) die Nitroimidazolverbindung in einem Bereich von 0,1-2% vorliegt und der Rest der Träger ist, oder
(d) die Nitroimidazolverbindung in einem Bereich von 0,75-2% vorliegt und der Rest der Träger ist,
und/oder wobei (i) die Nitroimidazolverbindung aus Metronidazol, Nimoranzol, Tinidazol, Ordinidazol, Secnidazol und Carnidazol ausgewählt ist oder (ii) die Nitroimidazolverbindung Metronidazol ist.

10. Verfahren zur Herstellung einer pharmazeutischen Zusammensetzung, umfassend:
Kombinieren einer zur Behandlung von Blepharitis und Blepharokonjunktivitis in einem Tier oder einem Menschen wirksamen Menge an Nitroimidazolverbindung mit einem Träger für die Nitroimidazolverbindung, wobei der Träger zum lokalen Aufbringen auf okuläre Gewebe geeignet ist und eine künstliche hypertone, isotone oder hypotone Tränenflüssigkeitszusammensetzung umfasst.

11. Verfahren nach Anspruch 10, wobei die künstliche Tränenflüssigkeitszusammensetzung einen Celluloseether oder ein anderes Cellulosederivat umfasst.

12. Verfahren nach Anspruch 11, wobei das Cellulosederivat Celluloseether ist und der Celluloseether zu etwa 1 Gew.-% im künstlichen Tränenflüssigkeitsträger vorliegt.

13. Verfahren nach Anspruch 11 oder 12, wobei das Cellulosederivat aus Methylcellulose, Hydroxypropylmethylcellulose und Hydroxyethylcellulose ausgewählt ist.

14. Verfahren nach Anspruch 13, wobei das Cellulosederivat Methylcellulose umfasst.

15. Verfahren nach einem der Ansprüche 10 bis 14, wobei die künstliche Tränenflüssigkeitszusammensetzung Polyvinylalkohol oder Polyvinylalkohol in einer Menge von etwa 1,4 Gew.-% des künstlichen Tränenflüssigkeitsträgers umfasst.

16. Verfahren zur Herstellung einer pharmazeutischen Zusammensetzung, umfassend Kombinieren einer zur Behandlung von Blepharitis und Blepharokonjunktivitis in einem Tier oder einem Menschen wirksamen Menge an Nitroimidazolverbindung mit einem von
(i) einem hypotonen Träger für die Nitroimidazolverbindung, wobei der Träger zum lokalen Aufbringen auf okuläre Gewebe geeignet ist,
(ii) einem hypertonen Träger für die Nitroimidazolverbindung, wobei der Träger zum lokalen Aufbringen auf okuläre Gewebe geeignet ist,
(iii) einem isotonen Träger für die Nitroimidazolverbindung, wobei der Träger zum lokalen Aufbringen auf okuläre Gewebe geeignet ist, oder
(iv) einem Träger für die Nitroimidazolverbindung, wobei der Träger zum lokalen Aufbringen auf okuläre Gewebe geeignet ist, wobei der Träger ein Shampoo ist, das zur Reinigung von Wimpern geeignet ist.

17. Verfahren zur Herstellung einer Augentropfenzubereitung, eines Wimpernshampoos, einer Augenwasch- oder Kontaktlinsenlösung, umfassend Kombinieren einer Nitroimidazolverbindung in einer Menge, die zur Behandlung von Blepharitis und Blepharokonjunktivitis wirksam ist, mit einem Träger für die Nitroimidazolverbindung, der zur Herstellung einer Augentropfenzubereitung, eines Wimpernshampoos, einer Augenwasch- oder Kontaktilnsenlösung geeignet ist.

18. Verfahren nach Anspruch 17, weiter umfassend das/die spezielle(n) in Anspruch 4 und/oder einem der Ansprüche 5 und 6 angegebene(n) Merkmal(e).

19. Ein Produkt, umfassend:
(i) eine pharmazeutische Zubereitung, die lokal an okuläre Gewebe verabreichbar ist und eine Nitroimidazolverbindung in einer Menge umfasst, die zur Behandlung von Blepharitis und Blepharokonjunktivitis wirksam ist, sowie einen ophthalmisch verträglichen Träger, und
(ii) als nicht-pharmazeutische Komponenten eine Flasche und einen Augentropfen-Applikator.

20. Produkt nach Anspruch 19, weiter umfassend das/die spezielle(n) in Anspruch 4 und/oder einem der Ansprüche 5 und 6 angegebene(n) Merkmal(e).

## Revendications (Revendications pour l'(les) Etat(s) contractant(s) suivant(s): AT, BE, CH, LI, DE, DK, FR, GB, IT, LU, NL, SE)

1. Utilisation d'un composé nitroimidazole pour la fabrication d'une préparation locale pour les yeux pour le traitement de la blépharite et/ou la blépharoconjonctivite et/ou la rosacée oculaire par application locale de la préparation sur les tissus oculaires.

2. Utilisation selon la revendication 1, dans laquelle le médicament comprend un véhicule ophtalmique convenant à une application locale aux tissus oculaires, ainsi que le composé nitroimidazole, et le véhicule comprend :
(a) un véhicule à base de vaseline ou une autre base de pommade convenant à une application directe sur l'oeil,
(b) un gel soluble dans l'eau, ou
(c) un liquide convenant à l'application sur l'oeil sous forme de gouttes.

3. Utilisation selon la revendication 1, dans laquelle le médicament comprend un véhicule ophtalmique convenant à une application locale aux tissus oculaires, ainsi que le composé nitroimidazole, et le véhicule comprend :
(a) une base liquide non aqueuse,
(b) un base d'huile minérale,
(c) un mélange d'une huile minérale et de vaseline,
(d) une suspension de particules solides dans un liquide, ou
(e) une suspension d'Amberlite ou d'une autre résine échangeuse d'ions dans de l'eau.

4. Utilisation selon l'une quelconque des revendications 1 à 3, dans laquelle
(a) ledit composé nitroimidazole est présent dans une gamme de 0,1 à 1 % et le complément est ledit véhicule,
(b) ledit composé nitroimidazole est présent dans une gamme de 0,75 à 1 % et le complément est ledit véhicule,
(c) ledit composé nitroimidazole est présent dans une gamme de 0,1 à 2 % et le complément est ledit véhicule, ou
(d) ledit composé nitroimidazole est présent dans une gamme de 0,75 à 2 % et le complément est ledit véhicule.

5. Utilisation selon l'une quelconque des revendications 1 à 4, dans laquelle le composé nitroimidazole est choisi parmi le métronidazole, le nimoranzole, le timidazole, l'ordinidazole, le secnidazole et le carnidazole.

6. Utilisation selon la revendication 5, dans laquelle le nitroimidazole est le métronidazole.

7. Procédé de fabrication d'une préparation locale pour les yeux destinée à une utilisation pour le traitement de la blépharite et/ou de la blépharoconjonctivite et/ou de la rosacée oculaire par application locale de la préparation sur les tissus oculaires comprenant la fabrication d'un composé nitroimidazole dans une préparation locale pour les yeux pour une telle utilisation.

8. Procédé selon la revendication 7, dans lequel le véhicule ophtalmique convenant à une application locale sur les tissus oculaires est transformé en un médicament avec le composé nitroimidazole, et le véhicule comprend :
(a) un véhicule à base de vaseline ou une autre base de pommade convenant à une application directe sur l'oeil,
(b) un gel soluble dans l'eau,
(c) un liquide convenant à l'application sur l'oeil sous forme de gouttes,
(d) une base liquide non aqueuse,
(e) un base d'huile minérale,
(f) un mélange d'une huile minérale et de vaseline,
(g) une suspension de particules solides dans un liquide, ou
(h) une suspension d'Amberlite ou d'une autre résine échangeuse d'ions dans de l'eau.

9. Procédé selon la revendication 7 ou la revendication 8, dans lequel
(a) ledit composé nitroimidazole est présent dans une gamme de 0,1 à 1 % et le complément est ledit véhicule,
(b) ledit composé nitroimidazole est présent dans une gamme de 0,75 à 1 % et le complément est ledit véhicule,
(c) ledit composé nitroimidazole est présent dans une gamme de 0,1 à 2 % et le complément est ledit véhicule, ou
(d) ledit composé nitroimidazole est présent dans une gamme de 0,75 à 2 % et le complément est ledit véhicule,
et/ou dans lequel (i) le composé nitroimidazole est choisi parmi le métronidazole, le nimoranzole, le timidazole, l'ordinidazole, le secnidazole et le carnidazole, ou (ii) le composé nitroimidazole est le métronidazole.

10. Composition pharmaceutique, comprenant :
une quantité de composé nitroimidazole efficace pour traiter la blépharite et la blépharoconjonctivite chez un patient animal ou humain ; et
un véhicule pour ledit composé nitroimidazole, le véhicule convenant à une application locale aux tissus oculaires et comprenant une composition de larmes artificielles qui est hypertonique, isotonique ou hypotonique.

11. Composition pharmaceutique selon la revendication 10, dans laquelle la composition de larmes artificielles est un éther de cellulose ou un autre dérivé de cellulose.

12. Composition pharmaceutique selon la revendication 11, dans laquelle le dérivé de cellulose est l'éther de cellulose et l'éther de cellulose est présent à raison d'environ 1 % en poids du véhicule de larmes artificielles.

13. Composition pharmaceutique selon la revendication 11 ou la revendication 12, dans laquelle le dérivé de cellulose est choisi parmi la méthylcellulose, l'hydroxypropylméthylcellulose et l'hydroxyéthylcellulose.

14. Composition pharmaceutique selon la revendication 13, dans laquelle le dérivé de cellulose comprend la méthylcellulose.

15. Composition pharmaceutique selon l'une quelconque des revendications 10 à 14, dans laquelle la composition de larmes pour les yeux comprend du poly(alcool vinylique).

16. Composition pharmaceutique selon la revendication 15, dans laquelle le poly(alcool vinylique) est présent à raison d'environ 1,4 % en poids du véhicule de larmes artificielles.

17. Produit pharmaceutique comprenant une composition adaptée à une application locale aux tissus oculaires à l'exclusion d'une application locale non oculaire, comprenant :
une quantité de composé nitroimidazole efficace pour traiter la blépharite et la blépharoconjonctivite chez un patient animal ou humain ; et
un véhicule ophtalmique pour ledit composé nitroimidazole, le véhicule convenant à une application locale aux tissus oculaires.

18. Préparation de gouttes pour les yeux, shampooing pour les cils, lavage oculaire ou solution pour lentilles de contact, comprenant une quantité de composé nitroimidazole efficace pour traiter la blépharite et la blépharoconjonctivite chez un patient animal ou humain.

19. Composition selon la revendication 17 ou préparation, shampooing, lavage oculaire ou solution pour lentilles de contact selon la revendication 18, qui comprend en outre la ou les caractéristiques spécifiques citées dans la revendication 4 et/ou dans l'une quelconque des revendications 5 et 6.

20. Produit destiné à une utilisation pour le traitement de la blépharite et de la blépharoconjonctivite, comprenant :
une préparation qui est susceptible d'être administrée localement aux tissus oculaires et qui comprend un composé nitroimidazole et un véhicule acceptable au plan ophtalmique,
un flacon, et
un applicateur de gouttes pour les yeux.

21. Produit selon la revendication 20, dans lequel la préparation comprend en outre la ou les caractéristiques spécifiques citées dans la revendication 4 et/ou dans l'une quelconque des revendications 5 et 6.

## Revendications (Revendications pour l'(les) Etat(s) contractant(s) suivant(s): ES)

1. Utilisation d'un composé nitroimidazole pour la fabrication d'une préparation locale pour les yeux pour le traitement de la blépharite et/ou la blépharoconjonctivite et/ou la rosacée oculaire par application locale de la préparation sur les tissus oculaires.

2. Utilisation selon la revendication 1, dans laquelle le médicament comprend un véhicule ophtalmique convenant à une application locale aux tissus oculaires, ainsi que le composé nitroimidazole, et le véhicule comprend :
(a) un véhicule à base de vaseline ou une autre base de pommade convenant à une application directe sur l'oeil,
(b) un gel soluble dans l'eau, ou
(c) un liquide convenant à l'application sur l'oeil sous forme de gouttes.

3. Utilisation selon la revendication 1, dans laquelle le médicament comprend un véhicule ophtalmique convenant à une application locale aux tissus oculaires, ainsi que le composé nitroimidazole, et le véhicule comprend :
(a) une base liquide non aqueuse,
(b) un base d'huile minérale,
(c) un mélange a une huile minérale et de vaseline,
(d) une suspension de particules solides dans un liquide, ou
(e) une suspension d'Amberlite ou d'une autre résine échangeuse d'ions dans de l'eau.

4. Utilisation selon l'une quelconque des revendications 1 à 3, dans laquelle
(a) ledit composé nitroimidazole est présent dans une gamme de 0,1 à 1 % et le complément est ledit véhicule,
(b) ledit composé nitroimidazole est présent dans une gamme de 0,75 à 1 % et le complément est ledit véhicule,
(c) ledit composé nitroimidazole est présent dans une gamme de 0,1 à 2 % et le complément est ledit véhicule, ou
(d) ledit composé nitroimidazole est présent dans une gamme de 0,75 à 2 % et le complément est ledit véhicule.

5. Utilisation selon l'une quelconque des revendications 1 à 4, dans laquelle le composé nitroimidazole est choisi parmi le métronidazole, le nimoranzole, le timidazole, l'ordinidazole, le secnidazole et le carnidazole.

6. Utilisation selon la revendication 5, dans laquelle le nitroimidazole est le métronidazole.

7. Procédé de fabrication d'une préparation locale pour les yeux destinée à une utilisation pour le traitement de la blépharite et/ou de la blépharoconjonctivite et/ou de la rosacée oculaire par application locale de la préparation sur les tissus oculaires comprenant la fabrication d'un composé nitroimidazole dans une préparation locale pour les yeux pour une telle utilisation.

8. Procédé selon la revendication 7, dans lequel le véhicule ophtalmique convenant à une application locale sur les tissus oculaires est transformé en un médicament avec le composé nitroimidazole, et le véhicule comprend :
(a) un véhicule à base de vaseline ou une autre base de pommade convenant à une application directe sur l'oeil,
(b) un gel soluble dans l'eau,
(c) un liquide convenant à l'application sur l'oeil sous forme de gouttes,
(d) une base liquide non aqueuse,
(e) un base d'huile minérale,
(f) un mélange d'une huile minérale et de vaseline,
(g) une suspension de particules solides dans un liquide, ou
(h) une suspension d'Amberlite ou d'une autre résine échangeuse d'ions dans de l'eau.

9. Procédé selon la revendication 7 ou la revendication 8, dans lequel
(a) ledit composé nitroimidazole est présent dans une gamme de 0,1 à 1 % et le complément est ledit véhicule,
(b) ledit composé nitroimidazole est présent dans une gamme de 0,75 à 1 % et le complément est ledit véhicule,
(c) ledit composé nitroimidazole est présent dans une gamme de 0,1 à 2 % et le complément est ledit véhicule,
(d) ledit composé nitroimidazole est présent dans une gamme de 0,75 à 2 % et le complément est ledit véhicule,
et/ou dans lequel (i) le composé nitroimidazole est choisi parmi le métronidazole, le nimoranzole, le timidazole, l'ordinidazole, le secnidazole et le carnidazole, ou (ii) le composé nitroimidazole est le métronidazole.

10. Procédé de préparation d'une composition pharmaceutique, comprenant :
la combinaison d'une quantité de composé nitroimidazole efficace pour traiter la blépharite et la blépharoconjonctivite chez un patient animal ou humain avec un véhicule pour le composé nitroimidazole, le véhicule convenant à une application locale aux tissus oculaires et comprenant une composition de larmes artificielles qui est hypertonique, isotonique ou hypotonique.

11. Procédé selon la revendication 10, dans lequel la composition de larmes artificielles comprend un éther de cellulose ou un autre dérivé de cellulose.

12. Procédé selon la revendication 11, dans lequel le dérivé de cellulose est l'éther de cellulose et l'éther de cellulose est présent à raison d'environ 1 % en poids du véhicule de larmes artificielles.

13. Procédé selon la revendication 11 ou la revendication 12, dans lequel le dérivé de cellulose est choisi parmi la méthylcellulose, l,hydroxypropylméthylcellulose et l'hydroxyéthylcellulose.

14. Procédé selon la revendication 13, dans lequel le dérivé de cellulose comprend la méthylcellulose.

15. Procédé selon l'une quelconque des revendications 10 à 14, dans laquelle la composition de larmes pour les yeux comprend du poly(alcool vinylique) ou comprend du poly(alcool vinylique) en une quantité d'environ 1,4 % en poids du véhicule de larmes artificielles.

16. Procédé de préparation d'une composition pharmaceutique, comprenant :
la combinaison d'une quantité de composé nitroimidazole efficace pour traiter la blépharite et la blépharoconjonctivite chez un patient animal ou humain avec l'un des véhicules parmi
(i) un véhicule hypotonique pour le composé nitroimidazole, le véhicule convenant à une application locale aux tissus oculaires,
(ii) un véhicule hypertonique pour le composé nitroimidazole, le véhicule convenant à une application locale aux tissus oculaires,
(iii) un véhicule isotonique pour le composé nitroimidazole, le véhicule convenant à une application locale aux tissus oculaires, ou
(iv) un véhicule pour ledit composé nitroimidazole, le véhicule convenant à une application locale aux tissus oculaires, dans lequel ledit véhicule est un shampooing convenant au nettoyage des cils.

17. Procédé de fabrication d'une préparation de gouttes pour les yeux, d'un shampooing pour les cils, d'un lavage oculaire ou d'une solution pour lentilles de contact, comprenant la combinaison d'une quantité de composé nitroimidazole efficace pour traiter la blépharite et la blépharoconjonctivite avec un véhicule pour le composé nitroimidazole convenant à la fabrication respectivement d'une préparation de gouttes pour les yeux, d'un shampooing pour les cils, d'un lavage oculaire ou d'une solution pour lentilles de contact.

18. Procédé selon la revendication 17, qui comprend en outre la ou les caractéristiques spécifiques citées dans la revendication 4 et/ou dans l'une quelconque des revendications 5 et 6.

19. Produit comprenant :
(i) une préparation pharmaceutique qui est susceptible d'être administrée localement aux tissus oculaires et qui comprend un composé nitroimidazole en une quantité efficace pour le traitement de la blépharite et la blépharoconjonctivite et un véhicule acceptable au plan ophtalmique,
(ii) en tant que composants non pharmaceutiques, un flacon, et un applicateur de gouttes pour les yeux.

20. Produit selon la revendication 19, qui comprend en outre la ou les caractéristiques spécifiques citées dans la revendication 4 et/ou dans l'une quelconque des revendications 5 et 6.
